(19) Europäisches Patentamt
European Patent Office
Office européen des brevets

(11) EP 0 672 031 B1

(12) EUROPEAN PATENT SPECIFICATION

(45) Date of publication and mention of the grant of the patent: **12.03.2003 Bulletin 2003/11**

(21) Application number: **94900390.9**

(22) Date of filing: **29.10.1993**

(51) Int Cl.[7]: **C07C 65/21**, A61K 31/085, A61K 31/19, A61K 31/235, C07C 69/76, C07C 205/35, C07C 205/57, C07C 311/02, C07C 311/51, C07C 235/42, C07C 235/34

(86) International application number: **PCT/US93/10228**

(87) International publication number: **WO 94/012461 (09.06.1994 Gazette 1994/13)**

(54) **CATECHOL DIETHERS AS SELECTIVE PDE IV INHIBITORS**

CATHECOLDIETHER ALS SELEKTIVE PDE IV HEMMUNGSMITTEL

DIETHERS DE PYROCATECHINE UTILISES COMME INHIBITEURS SELECTIFS DE LA PDE IV

(84) Designated Contracting States:
**AT BE CH DE DK ES FR GB GR IE IT LI LU NL PT SE**

(30) Priority: **02.12.1992 US 984408**

(43) Date of publication of application: **20.09.1995 Bulletin 1995/38**

(73) Proprietor: **PFIZER INC.**
**New York, N.Y. 10017-5755 (US)**

(72) Inventors:
- **DUPLANTIER, Allen, J.**
  **Ledyard, CT 06339 (US)**
- **EGGLER, James, F.**
  **Stonington, CT 06378 (US)**
- **MARFAT, Anthony**
  **Mystic, CT 06355 (US)**
- **MASAMUNE, Hiroko**
  **Noank, CT 06340 (US)**

(74) Representative: **Hirsch, Denise Marie**
**Pfizer Global Research and Development**
**Fresnes Laboratories**
**3-9, rue de la Loge**
**B.P. 100**
**94265 Fresnes Cedex (FR)**

(56) References cited:

| | |
|---|---|
| **EP-A- 0 428 302** | **EP-A- 0 428 313** |
| **EP-A- 0 511 865** | **WO-A-84/04521** |
| **WO-A-87/06576** | **WO-A-92/06085** |
| **WO-A-92/06963** | **WO-A-92/19594** |

- **CHEMICAL ABSTRACTS, vol. 115, no. 15, 1991, Columbus, Ohio, US; abstract no. 158680z, G. WANG ET AL. 'A convinient method for the conversion of alpha-tetralones to aryl acetates' page 891 ; & SYNTH. COMMUN. 1991, 21(8-9), 989-96**
- **CHEMICAL ABSTRACTS, vol. 112, no. 19, 1990, Columbus, Ohio, US; abstract no. 172319h, M. AKASU ET AL. 'Anticancer agents containing 1-benzylisoquinolines' page 80 ; & JP,A,01 233 221 (KAKEN) 19 September 1989**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 672 031 B1

## Description

### Background of the invention

This invention relates to a series of 4-substituted catechol diether compounds which are selective inhibitors of phosphodiesterase (PDE) type IV and as such are useful in the treatment of AIDS, asthma, arthritis, bronchitis, chronte obstructive airways disease, psoriasis, allergic rhinitis, dermatitis and other inflammatory diseases.

This invention also relates to the pharmaceutically acceptable salts of said compounds; to the use of such compounds in the treatment of inflammatory conditions in mammals, especially humans; and to pharmaceutical compositions useful therefor.

The "inflammatory conditions" which can be treated according to this invention Include, but are not limited to, chronic obstructive pulmonary disease, shock, atopic dermatitis, bronchitis, rheumatoid arthritis and osteoarthritis.

Since the recognition that cyclic AMP is an intracellular second messenger (E. W. Sutherland, and T. W. Rall, Pharmacol. Rev., 1960, 12, 265), inhibition of the phosphodiesterases have been a target for modulation and, accordingly, therapeutic intervention In a range of disease processes. More recently, distinct classes of PDE have been recognized (J. A. Beavo and D. H. Reifsnyder, TiPS, 1990, 11, 150), and their selective Inhibition has led to improved drug therapy (C. D. Nicholson, R. A. Challiss and M. Shahid, TiPS, 1991, 12, 19). More particularly, it has been recognized that inhibition of PDE type IV can lead to inhibition of inflammatory mediator release (M. W. Verghese *et al.*, J. Mol. Cell Cardiol., 1989, 12 (Suppl. II), S 61) and airway smooth muscle relaxation (T. J. Torphy in Directions for New Anti-Asthma Drugs, eds S. R. O'Donnell and C. G. A. Persson, 1988, 37, Birkhauser-Veriag). Thus, compounds that inhibit PDE type IV, but which have poor activity against other PDE types, would inhibit the release of inflammatory mediators and relax airway smooth muscle without causing cardiovascular effects or antiplatelet effects.

Certain pyrimidone compounds have been disclosed to be useful as antidepressants by Saccomano et al., in European Patent Application EPO 247 725 A2. The same pyrimidone compounds have been disclosed to be useful against asthma and certain skin disorders In International Patent Application No. PCT/US90/02162. filed April 20, 1990.

### Summary of the Invention

This invention is concerned with a series of 4-substituted catechol diether compounds and to the pharmaceutically acceptable salts of such compounds. These new compounds possess antlinflammatory activity in mammals, especially humans.

The compounds of the present invention are of the formula (I)

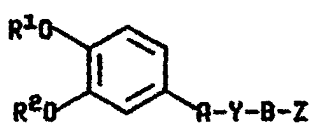 ,

(I)

the racemic-diastereomeric mixtures and optical isomers of compounds of formula land the pharmaceutically acceptable salts thereof wherein

$R^1$ is selected from the group consisting of methyl, ethyl, difluoromethyl and trifluoromethyl;

$R^2$ is selected from the group consisting of $(C_1\text{-}C_6)$alkyl, alkoxyalkyl having 3 to 7 carbons in the alkoxy portion and 2 to 4 carbons in the alkyl portion, phenoxyalkyl having 2 to 6 carbons in the alkyl portion, $(C_3\text{-}C_7)$cycloalkyl, $(C_6\text{-}C_8)$ polycycloalkyl, phenylalkyl having 1 to 8 carbons in the alkyl portion, phenylaminoalkyl having 2 to 6 carbons in the alkyl portion and the amino may be optionally substituted with $(C_1\text{-}C_4)$ alkyl and indanyl,

where the alkyl portion of said alkyl, phenoxyalkyl, cycloalkyl, polycycloalkyl, phenylalkyl and indanyl may optionally be substituted with one or more fluorine atoms, -OH or $(C_1\text{-}C_4)$alkoxy,

and the aryl portion of said phenylalkyl, phenoxyalkyl and indanyl may optionally be substituted with $(C_1\text{-}C_4)$alkyl, $(C_1\text{-}C_4)$alkoxy or halogen;

A and B are independently selected from the group consisting of a covalent bond, optionally substituted $(C_1\text{-}C_5)$alkylene, optionally substituted $(C_2\text{-}C_5)$alkenyl and optionally substituted phenylene,

where said optionally substituted alkylene may be monosubstituted and each substituent is selected from the group consisting of oxo, $(C_1\text{-}C_4)$alkoxy, $CO_2R^6$ and hydroxy,

said optionally substituted alkenyl may be monosubstituted with $(C_1\text{-}C_4)$alkoxy or $CO_2R^6$, and

said optionally substituted phenylene may be monosubstituted with $(C_1\text{-}C_4)$alkoxy, $CO_2R^6$ or hydroxy, wherein $R^6$ is hydrogen or $(C_1\text{-}C_4)$alkyl;

Y is selected from the group consisting of a covalent bond, O, $NR^6$ and S wherein $R^6$ is as defined above;

Z is selected from the group consisting of

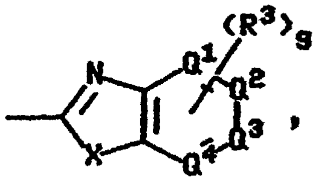 , 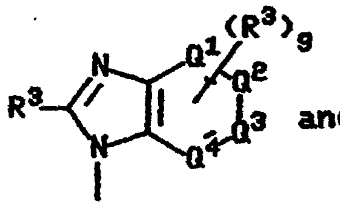 and 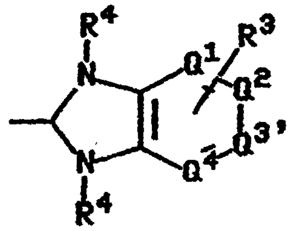 ,

where $Q^1$, $Q^2$, $Q^3$, and $Q^4$ are CH ;

X is $NR^4$ ;

g is an integer from 1 to 4;

each $R^3$ is independently selected from the group consisting of hydrogen, halogen, $(C_1\text{-}C_6)$alkyl, $CH(R^7)CO_2R^4$, $(C_1\text{-}C_6)$ alkoxy, $CO_2R^4$, $CONR^4R^5$, CONHOH, $CH_2NR^4R^5$, $NR^4R^5$, nitro, hydroxy, CN, $SO_3H$, phenylalkyl having 1 to 4 carbons in the alkyl portion, $SO_2NR^4R^5$, $N(SO_2R^6)_2$ and $NHSO_2R^8$,

where $R^4$ for each occurrence is independently selected from the group consisting of hydrogen, $(C_1\text{-}C_6)$alkyl, phenyl optionally substituted with $(C_1\text{-}C_4)$alkyl or halogen, $CH(R^7)CO_2R^6$, $(C_3\text{-}C_7)$cycloalkyl, phenylalkyl having 1 to 4 carbons in the alkyl portion and dialkylaminoalkyl having a total of 5 carbons In the dialkylamino portion and having 2 to 5 carbons in the alkyl portion where $R^6$ is as defined above,

$R^5$ for each occurrence is independently selected from the group consisting of hydrogen, $(C_1\text{-}C_6)$alkyl, $(C_3\text{-}C_7)$ cycloalkyl, phenylalkyl having 1 to 4 carbons in the alkyl portion, phenyl, pyridyl, pyrimidyl, thiazolyl and oxazolyl,

or $R^4$ and $R^5$ are taken together with the nitrogen to which they are attached and form an optionally substituted saturated or unsaturated 5- or 6-membered ring, a saturated or unsaturated 6-membered heterocyclic ring containing two heteroatoms, or a quinoline ring optionally substituted with fluoro,

where said optionally substituted saturated or unsaturated 5- or 6-membered ring may be mono- or di-substituted and each substituent is independently selected from the group consisting of alkyl having 1 to 4 carbons, $CO_2R^7$ wherein $R^7$ is as defined below, $CONH_2$, $CON(CH_3)_2$, oxo, hydroxy, $NH_2$ and $N(CH_3)_2$, and said saturated or unsaturated 6-membered heterocyclic ring containing two heteroatoms has the second heteroatom selected from the group consisting of O, S, NH, $NCH_3$, $NCOCH_3$ and $NCH_2Ph$;

$R^7$ for each occurrence is independently selected from the group consisting of hydrogen and $(C_1\text{-}C_4)$alkyl;

and $R^8$ is selected from the group consisting of $(C_1\text{-}C_6)$alkyl, $(C_3\text{-}C_7)$cycloalkyl, phenyl and phenylalkyl having 1 to 4 carbons in the alkyl portion.

As used throughout this specification and the appendant claims, the terms "alkyl" and "alkoxy" include both straight chain and branched groups; the term "halogen" includes fluoro, chloro and bromo; and the symbol "Ph" in the term "$NCH_2Ph$" means phenyl.

Those members of the substituent Z which are blcyclic are attached to the remainder of the compound of formula (I) through the ring of the 2 substituent in which the bond is drawn.

A preferred group of compounds is that group of compounds of formula (I), above, wherein

$R^1$ is selected from the group consisting of methyl and difluoromethyl;

$R^2$ is selected from the group consisting of $(C_3\text{-}C_7)$cycloalkyl, $(C_6\text{-}C_9)$polycycloalkyl, phenylalkyl having 1 to 8 carbons in the alkyl portion and phenoxyalkyl having 2 to 6 carbons in the alkyl portion;

A is selected from the group consisting of a covalent bond, $(C_1\text{-}C_5)$-alkylene and $(C_2\text{-}C_5)$alkenyl;

B is selected from the group consisting of a covalent bond, phenylene optionally substituted with $(C_1\text{-}C_4)$alkoxy, $(C_1\text{-}C_5)$ alkylene and $(C_2\text{-}C_5)$alkenyl;

Y is selected from the group consisting of a covalent bond, O and $NR^6$;

Z is selected from the group consisting of

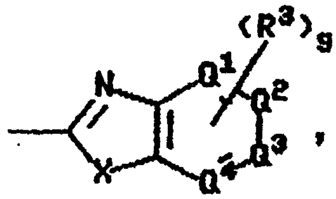 , 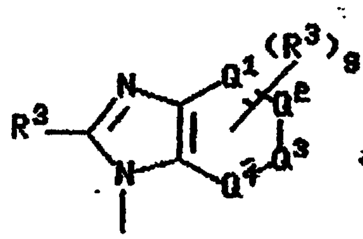 and 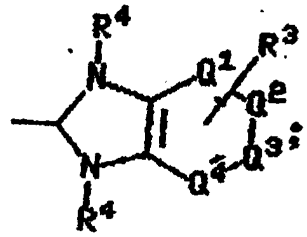 ;

each $R^3$ is independently selected from the group consisting of hydrogen, halogen, $(C_1\text{-}C_6)$alkyl, $CH(R^7)CO_2R^4$, $(C_1\text{-}C_6)$alkoxy, $CO_2R^4$, $CONR^4R^5$, nitro, hydroxy, $N(SO_2R^6)_2$ and $NHSO_2R^8$,
where $R^4$ for each occurrence is independently selected from the group consisting of hydrogen and $(C_1\text{-}C_6)$alkyl and $R^5$ is selected from the group consisting of hydrogen and $(C_1\text{-}C_6)$alkyl.

A more preferred group of compounds is that group of compounds of formula (I), above, wherein $R^1$ is selected from the group consisting of methyl and difluoromethyl;
$R^2$ is selected from the group consisting of $(C_3\text{-}C_7)$cycloalkyl, $(C_6\text{-}C_9)$polycycloalkyl and phenylalkyl having 1 to 8 carbons in the alkyl portion;
A is selected from the group consisting of a covalent bond and methylene;
B is selected from the group consisting of a covalent bond, methylene and phenylene;
Y is selected from the group consisting of a covalent bond and O; and
Z is selected from the group consisting of

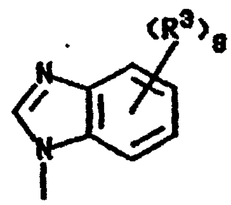 and 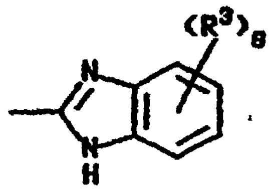 .

Another group of preferred compounds is that group of compounds of formula (I), above, wherein $R^1$ is selected from the group consisting of methyl and difluoromethyl; $R^2$ is selected from the group consisting of $(C_3\text{-}C_7)$cycloalkyl, $(C_6\text{-}C_9)$polycycloalkyl and phenylalkyl having 1 to 8 carbons in the alkyl portion; A, B and Y are a covalent bond; and Z is selected from the group consisting of

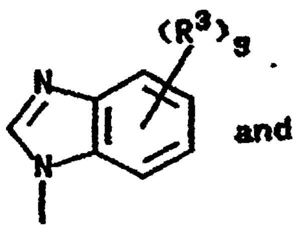 and 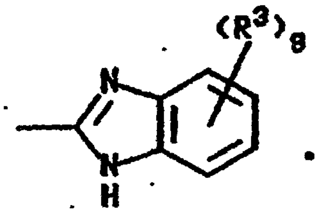 .

Furthermore, another group of preferred compounds is that group of compounds of formula (I), above, wherein $R^1$ is selected from the group consisting of methyl and difluoromethyl; $R^2$ is selected from the group consisting of $(C_3\text{-}C_7)$cycloalkyl, $(C_6\text{-}C_9)$polycycloalkyl, phenylalkyl having 1 to 8 carbons in the alkyl portion and phenoxyalkyl having 2 to 6 carbons in the alkyl portion; A is methylene; B is phenylene; Y is O and Z is selected from the group consisting of

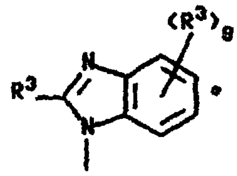

The starting materials and reagents required for the synthesis of the compounds of the present invention are readily available, either commercially, according to literature methods, or by methods exemplified in Preparations below.

Detailed Description of the Invention

The compounds of the invention can be prepared by a number of different processes according to the invention.

(a) The following procedure is used to synthesize compounds of the formula

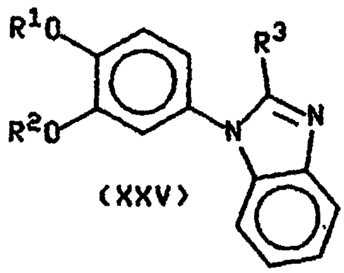

(XXV)

wherein $R^1$, $R^2$ and $R^3$ are as defined above for formula (I). About one equivalent of a compound of the formula

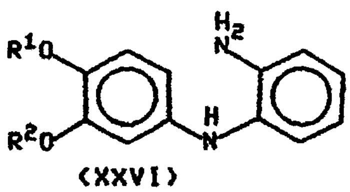 is

(XXVI)

mixed with ethyl formate and approximately 25 ml of formic acid and heated at about 100°C for about 18 hours. The solvent is evaporated and the residue chromatographed on silica gel to yield the desired benzimidazole derivatives of formula (XXV).

(b) Compounds having the general formula

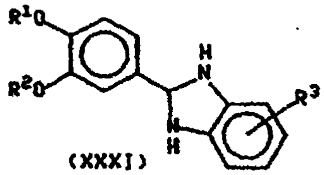

(XXXI)

wherein $R^1$, $R^2$ and $R^3$ are as defined above for formula (I), are synthesized by the following general method. A compound of the general formula (II) is mixed with an

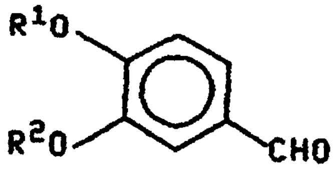

(II)

appropriate compound of the general formula

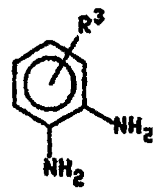

(XXXII)

and the mixture heated to about 120°C for about 1 to 6 hours. The resulting residue is chromatographed on silica gel to yield the desired derivative of formula (XXXI).

The synthetic methods outlined above in methods a to b together with the following Examples describe methods which were and can be employed to prepare the compounds of this invention.

The ability of the compounds or the pharmaceutically acceptable salts thereof to inhibit phosphodiesterase IV ($PDE_4$) and, consequently, demonstrate their effectiveness for treating inflammatory diseases is shown by the following in vitro assay.

BIOLOGICAL ASSAY

(Human lung $PDE_{IV}$)

Thirty to forty grams of human lung tissue is placed in 50 ml of pH 7.4 Tris/phenylmethylsulfonyl fluoride (PMSF)/sucrose buffer and homogenized using a Tekmar Tissumlzer® (Tekmar Co., 7143 Kemper Road, Cincinnati, Ohio 45249) at full speed for 30 seconds. The homogenate is centrifuged at 48,000 x g for 70 minutes at 4°C. The supematant is filtered twice through a 0.22 µm filter and applied to a Mono-Q FPLC column (Pharmacia LKB Biotechnology, 800 Centennial Avenue, Piscataway, New Jersey 08854) pre-equilibrated with pH 7.4 Tris/PMSF buffer. A flow rate of 1 ml/minute is used to apply the sample to the column, followed by a 2 ml/minute flow rate for subsequent washing and elution. Sample is eluted using an increasing, step-wise NaCl gradient in the pH 7.4 Tris/PMSF buffer. Eight ml fractions are collected. Fractions are assayed for specific $PDE_{IV}$ activity, determined by [$^3$H]cAMP hydrolysis and the ability of a known $PDE_{IV}$ inhibitor (e.g. rolipram) to inhibit that hydrolysis. Appropriate fractions are pooled, diluted with ethylene glycol (2 ml ethylene glycol/5 ml of enzyme prep) and stored at -20°C until use.

Compounds are dissolved in DMSO at a concentration of 10 mM and diluted 1:25 in water (400 µM compound, 4% DMSO). Further serial dilutions are made in 4% DMSO to achieve desired concentrations. Final DMSO concentration in assay tube is 1%. In duplicate the following are added, in order, to a 12 x 75 mm glass tube (all concentrations are given as final concentrations in assay tube).

i) 25 µl compound or DMSO (1%, for control and blank)
ii) 25 µl pH 7.5 Tris buffer
iii) [$^3$H]cAMP (1 µM)
iv) 25 µl $PDE_{IV}$ enzyme (for blank, enzyme is preincubated in boiling water for 5 minutes)

The reaction tubes are shaken and placed in a water bath (37°C) for 20 minutes, at which time the reaction

is stopped by placing the tubes in a boiling water bath for 4 minutes. Washing buffer (0.5 ml, 0.1M 4-(2-hydroxyethyl)-1-piperazineethanesulfonic acid (HEPES)/0.1M NaCl, pH 8.5) is added to each tube on an ice bath. The contents of each tube are applied to an Affi-Gel 601 column (Biorad Laboratories, P.O. Box 1229, 85A Marcus Drive, Melville, New York 11747) (boronate affinity gel, 1 ml bed volume) previously equilibrated with washing buffer. [$^3$H]cAMP is washed with 2 x 6 ml washing buffer, and [$^3$H]5'AMP is then eluted with 4 ml of 0.25M acetic acid. After vortexing, 1 ml of the elution is added to 3 ml scintillation fluid in a suitable vial, vortexed and counted for [$^3$H].
% Inhibition is determined by the formula:

$$\% \text{ Inhibition} = 1 - \frac{\text{average cpm (test compound) - average cpm (blank)}}{\text{average cpm (control) - average cpm (blank)}}$$

$IC_{50}$ is defined as that concentration of compound which inhibits 50% of specific hydrolysis of [$^3$H]cAMP to [$^3$H]5'AMP.

Pharmaceutically-acceptable acid addition salts of the compounds of this invention include, but are not limited to, those formed with HCl, HBr, $HNO_3$, $H_2SO_4$. $H_3PO_4$, $CH_3SO_3H$, p-$CH_3C_6H_4SO_3H$, $CH_3CO_2H$, gluconic acid, tartaric acid, maleic acid and succinic acid. In the case of those compounds of the formula (I) which contain a further basic nitrogen. It will, of course, be possible to form diacid addition salts (e.g., the dihydrochloride) as well as the usual monoacid addition salt. Pharmaceutically-acceptable cationic salts of the compounds of this invention include, but are not limited to, those of sodium, potassium, calcium, magnesium, ammonium, N,N'-dibenzylethylenediamine, N-methylglucamine (meglumine), ethanolamine and diethanoiamine.

For administration to humans In the curative or prophylactic treatment of inflammatory conditions, oral dosages of the compounds are generally in the range of from 0.1-100 mg daily for an average adult patient (70 kg). Thus for a typical adult patient, individual tablets or capsules contain from 0.1 to 50 mg of active compound, in a suitable pharmaceutically acceptable vehicle or carrier. Dosages for intravenous administration are typically within the range of 0.1 to 10 mg per single dose as required. For Intranasal or inhaler administration, the dosage is generally formulated as a 0.1 to 1% (w/v) solution. In practice the physician will determine the actual dosage which will be most suitable for an individual patient and it will vary with the age, weight and response of the particular patient. The above dosages are exemplary of the average case but them can, of course, be individual instances where higher or lower dosage ranges are merited, and all such dosages are within the scope of this invention.

For human use, the compounds of the formula (I) can be administered alone, but will generally be administered in an admixture with a pharmaceutical diluent or carrier selected with regard to the intended route of administration and standard pharmaceutical practice. For example, they may be administered orally in the form of tablets containing such excipients as starch or lactose, or in capsules or ovales either alone or in admixture with excipients, or in the form of elixirs or suspensions containing flavoring or coloring agents. They may be injected parenterally; for example, intravenously, intramuscularly or subcutaneously. For parenteral administration, they are best used in the form of a sterile aqueous solution which may contain other substances; for example, enough salts or glucose to make the solution isotonic.

Thus in a further aspect the invention provides pharmaceutical compositions comprising a compound of the formula (I), or pharmaceutically acceptable salts thereof, together with a pharmaceutically acceptable diluent or carrier.

This invention also provides the use of a compound of the formula (I), or a pharmaceutically acceptable salt thereof in the manufacture of a medicament for the inhibition of phosphodiesterase (IV) in a mammal.

This invention further provides the use of a compound of the formula (I), or a pharmaceutically acceptable salt thereof in the manufacture of a medicament for the treatment of an inflammatory condition in a mammal.

Further still, this invention provides the use of a compound of the formula (I), or a pharmaceutically acceptable salt thereof in the manufacture of a medicament for the treatment of AIDS, asthma, arthritis, bronchitis, chronic obstructive pulmonary disease, psoriasis, allergic rhinitis, dermatitis or shock in a mammal.

The present invention is illustrated by the following Examples but is not limited to the details thereof.

EXAMPLE 1

Methyl 1-[4-[[3-(bicyclo[2.2.1]hept-2-yloxy)-4-methoxyphenyl]methoxyphenyl]-2-butyl-1H-benzimidazole-5-carboxylate

Diethylazodicarboxylate (201 µL, 1.28 mmol, 1.3 eq) was added to a mixture of (320 mg, 0.986 mmol, 1.0 eq) methyl 2-butyl-1-(4-hydroxyphenyl)-1H-benzimidazole-5-carboxylate, (269 mg, 1.08 mmol, 1.1 eq) (±)-3-exo-norbomyloxy-4-methoxybenzyl alcohol and (310 mg, 1.18 mmol, 1.2 eq) of triphenylphosphine in 10 ml of dry tetrahydrofuran at room temperature. The reaction mixture was stirred at room temperature for 18 hours, diluted with 300 ml of ethyl acetate and washed twice with 1N NaOH, once with $H_2O$, once with brine, dried over $MgSO_4$ and concentrated to give 0.7 g of an oil. Silica gel chromatography eluting with 35% ethyl acetate/hexane gave 298 mg, 54%, of an off-white foam. Mass Spectra: Calc'd for $C_{34}H_{38}N_2O_5$: 554.7. Found: 554.

EXAMPLE 2

1-[4-[[3-bicyclo[2.2.1]hept-2-yloxy)-4-methoxyphenyl]methoxy]phenyl-2-butyl-1H-benzimidazole-5-carboxiyic acid

Reaction of (260 mg, 0.469 mmol, 1.0 eq) methyl 1-[4-[[3-(bicyclo[2.2.1]hept-2-yloxy)-4-methoxyphenyl]methoxy]phenyl]-2-butyl-1H-benzimidazole-5-carboxylate,(2.3 ml, 2.3 mmol, 5.0 eq) substantially according to the procedure of Preparation 7 yielded the title compound, 178 mg, 70%, as a white solid. M.P.: 209-211°C. Elemental Analysis Calc'd for $C_{33}H_{38}N_2O_5$: Calc'd: C, 73.31; H, 6.71; N, 5.18. Found: C, 72.92; H, 6.74; N, 4.94.

EXAMPLE 3

1-[4-[[3-Bicyclo[2.2.1]hept-2-yloxy]-4-methoxy-phenyl]methoxy] phenyl-2-butyl-1H-benzimidazole

Diethylazodicarboxylate (90 µl, 0.680 mmol, 1.2 eq) was added to a mixture of (120 mg, 0.483 mmol, 1.0 eq) 2-butyl-3-(4-hydroxyphenyl)benzimidazole, (126mg, 0.507 mmol, 1.05 eq) (±)-3-exo-norbomyloxy-4-methoxybenzyl alcohol, and (139 mg, 0.531 mmol, 1.1 eq) triphenylphosphine in 10 ml anhydrous tetrahydrofuran. After 18 hours at room temperature, the reaction mixture was diluted with 200 ml of ethyl acetate, washed twice with 1N NaOH, once with $H_2O$, once with brine, dried over $MgSO_4$, concentrated in vacuo to give 0.2 g of an oil. Silica gel chromatography eluting with 40% ethyl acetate/hexane followed by recrystaltization from ether/hexane gave 66 mg, 28%, white crystals. M.P.: 134-136°C. Elemental Analysis Calc'd for $C_{32}H_{38}N_2O_3$: Calc'd: C, 77.39; H, 7.31; N, 6.64. Found: C, 77.08; H, 6.94; N, 5.43,

EXAMPLE 4

1-[3-(Cyclopentyloxy)-4-methoxyphenyl]-1H-benzimidazole-5-carboxylic acid

A mixture of (800 mg, 5.3 mmoles) cyclopentyl bromide, (1.6 g, 5.3 mmoles) methyl 1-(3-hydroxy-4-methoxyphenyl)-1H-benzimidazole-6-carboxylate and 251 mg of 60% sodium hydride in 10 ml of dimethylformamide was stirred at 100° for 30 minutes. The reaction was cooled, poured onto $H_2O$ and extracted with ethyl acetate. The ethyl acetate layer was dried and evaporated to give 2 g of crude product. Purification on silica gel with $CH_2Cl_2$ gave 1.1 g of methyl 1-(3-cyclopentyl-4-methoxyphenyl)-1H-benzimidazole-5-carboxylate. M.P.: 129-131°.

A solution of 500 mg of the above methyl ester in 20 ml of methanol containing 5 ml of 1N NaOH was heated on a steam bath for 30 minutes. The reaction was cooled and the methanol removed in vacuo. The residue was acidified with 1N HCl and the resulting solid was filtered and recrystallized from methanol to give 198 mg of product. M.P. >250°C.

EXAMPLE 5

2-{4-Methoxy-3-[4-(4-methoxyphenyl)-butoxy] phenyl}-2,3-dihydro-1H-benzimidazole-5-carboxylic acid

3-(4-(4-Methoxyphenyl)-butoxy)-4-methoxybenzaldehyde (2.8 g) and 3,4-diaminobenzoic add (1.4 g) were heated to about 120°C over 1 hour. The resulting residue was chromatographed on a 5 x 10 cm pad of silica gel eluting with ether to give 1.4 g of a beige solid which was recrystallized from 20 ml of methanol. M.P. 167-169°C. MS m/e 450 ($M^+$ + 1). Elemental analysis calculated for $C_{28}H_{28}O_5N_2$: C, 69.62; H, 6.29; N, 8.24. Found: C, 69.72; H, 6.70; N, 6.75.

EXAMPLES 6 to 16

Additional examples which were prepared according to the methods described and readily apparent to those skilled in the art are shown in the following table.

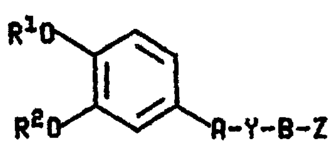

*CB = Covalent Bond

| Ex. # | $R^1$ | $R^2$ | A | Y | B | Z-$R^3$ | M.P.(°C) |
|---|---|---|---|---|---|---|---|
| 6 | $CH_3$ | 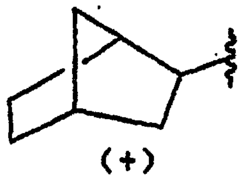 (+) | $-CH_2-$ | -O- | 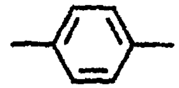 | 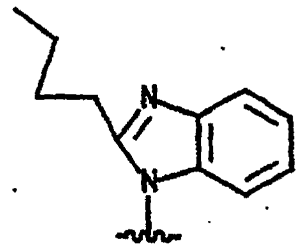 | 129-131 |
| 7 | $CH_3$ | 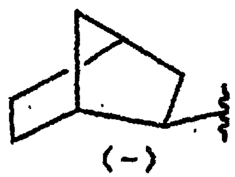 (−) | $-CH_2-$ | -O- | 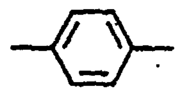 | 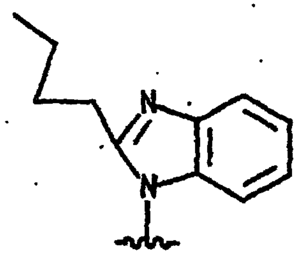 | 138-140 |

| Ex. # | $R^1$ | $R^2$ | A | Y | B | $Z-R^3$ | M.P.(°C) |
|---|---|---|---|---|---|---|---|
| 8 | $CH_3$ | 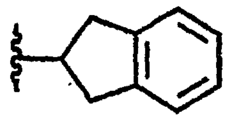 | C.B. | C.B. | C.B. | 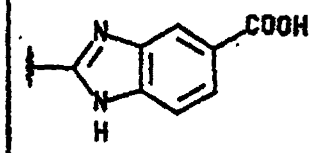 | 185-187 |
| 9 | $CH_3$ | 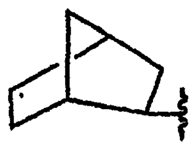 | C.B. | C.B. | C.B. | 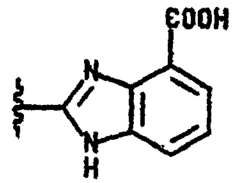 | 229-230 |

| Ex. # | $R^1$ | $R^2$ | A | Y | B | Z-$R^3$ | M.P.(°C) |
|---|---|---|---|---|---|---|---|
| 10 | $CH_3$ | 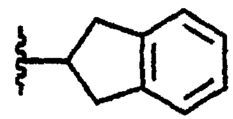 | C.B. | C.B. | C.B. | 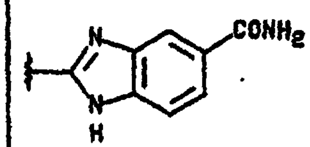 | 156-158 |

| Ex. # | $R^1$ | $R^2$ | A | Y | B | Z-$R^3$ | M.P.(°C) |
|---|---|---|---|---|---|---|---|
| 11 | $CH_3$ | 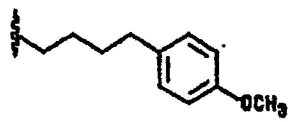 | C.B. | C.B. | C.B. | 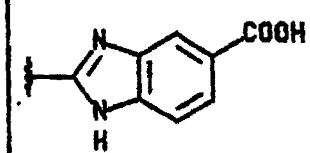 | 145-147 |
| 12 | $CH_3$ | 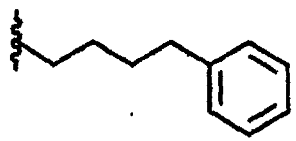 | C.B. | C.B. | C.B. | 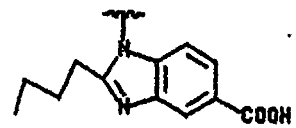 | 146-148 |

| Ex. # | $R^1$ | $R^2$ | A | Y | B | Z-$R^3$ | M.P.(°C) |
|---|---|---|---|---|---|---|---|
| 13 | $CH_3$ | 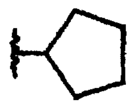 | C.B. | C.B. | C.B. | 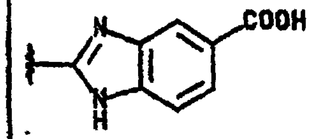 | 210 |
| 14 | $CH_3$ | 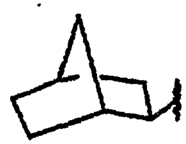 | C.B. | C.B. | C.B. | 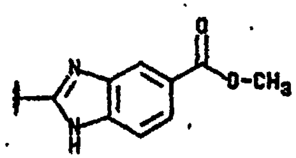 | 260-262 |

| Ex. # | $R^1$ | $R^2$ | A | Y | B | Z-$R^3$ | M.P.(°C) |
|---|---|---|---|---|---|---|---|
| 15 | $CH_3$ | 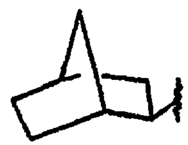 | C.B. | C.B. | C.B. | 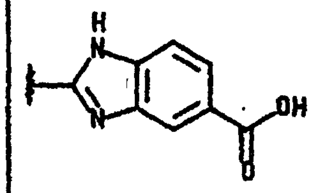 | 221-223 |

| Ex. # | $R^1$ | $R^2$ | A | Y | B | Z-$R^3$ | M.P.(°C) |
| --- | --- | --- | --- | --- | --- | --- | --- |
| 16 | $CH_3$ | 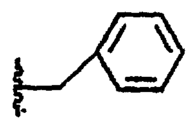 | C.B. | C.B. | C.B. | 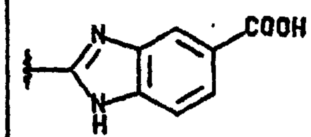 | 261-262 |

EXAMPLE 17

2-[4-Methoxy-3-(5-phenylpentyloxy)phenyl]-1H-benzimidazole-5-carboxylic acid

A solution of 4-methoxy-3-(5-phenylpentoxy)-benzoic acid (5.0 grams, 15.9 mmole) In thromyl chloride (20 ml) was heated to reflux for about 1 hour. The mixture was concentrated under reduced pressure and the residue was dissolved in dry THF (20 ml) and added to a stirred solution of methyl-3,4-diaminobenzoate in pyridine (20 ml) at about 0°C. After about 1 hour the mixture was concentrated under reduced pressure and 1NHCl (50 ml) was added. The resulting mixture was extracted with ethylacetate (100 ml x 3) and the combined organics were washed with water and brine, dried over sodium sulfate, filtered and concentrated under reduced pressure to give 7.3 grams of a red foam.

The above foam was suspended in phosphorous oxychloride (60 ml) and the mixture was heated to reflux for about 1 hour. The resulting homogeneous solution was concentrated under reduced pressure; water (100 ml) was added and the mixture was neutralized to pH 7-8 with 6N NaOH. To this was added saturated aqueous bicarb. (50 ml) and the mixture was extracted with ethyl acetate (3 x 100 ml). The combined organics were washed with water and brine, dried over sodium sulfate, filtered and concentrated under reduced pressure to give 6.81 grams of a tan solid.

The above solid was dissolved in a 1:1 mixture of ethanol (45 ml) and 1 N NaOH (45 ml). After stirring at reflux for about 1 hour the solution was concentrated under reduced pressure, dissolved in water (200 ml) and extracted with ether (50 ml). The aqueous layer was acidified to pH 1 with 6 N HCl and filtered. Recrystallization of the precipatate from methanol/isopropanol gave 4.4 grams of the title compound as a tan solid. MP 204-206°C; $^1$HNMR (250 MHz, DMSO-$d_6$) 1.44-1.53 (m, 2H), 1.60-1.72 (m, 2H), 1.75-1.88 (m, 2H), 2.62 (t, J=7.6 Hz, 2H), 3.87 (s, 3H), 4.10 (t, J=6.6 Hz, 2H), 7.15-7.31 (m, 6H), 7.74 (d, J=8.5 Hz, 1H), 7.87-7.97 (M, 3H), 8.21 (s, 1H). Anal. calc'd for $C_{26}H_{26}N_2O_4$•HCl: C, 66.88; H, 5.83; N, 6.00. Found: C, 67.20; H, 5.75; N, 6.10.

PREPARATION 1

3-(Bicyclo[2.2.1]hept-2-yloxy)-4-methoxybenzaldehyde

Diisopropylazodicarboxylate (7.8 ml, 39.5 mmol, 1.2 eq) was added neat to a 25° solution of (5.00 g, 32.9 mmol, 1.0 eq) 3-hydroxy-4-methoxybenzaldehyde (9.48 g, 36.1 mmol, 1.1 eq) triphenylphosphine, and (3.69 g, 32.9 mmol, 1.0 eq) (±)-endo-norbomeol in 100 ml of anhydrous tetrahydrofuran. After refluxing for 6 hours, the reaction mixture was poured into 1 liter of $H_2O$ and extracted twice with ethyl acetate. The ethyl acetate layers were combined and washed twice with $H_2O$, once with 1N NaOH, once with $H_2O$ and once with brine and then the solution was dried over anhydrous sodium sulfate. Filtration, concentration, and drying afforded 26.1 g of crude product, which was chromatographed on a silica gel column, eluting with 20% ethyl acetate-hexane to afford 5.68 g, 70% yield, of a yellow oil. IR($cm^{-1}$): 1680, 1580. NMR ($CDCl_3$): δ 9.82 (s, 1H), δ 4.27 (d, 1H). High resolution mass spectra (HRMS): 246.1300.

PREPARATION 2

Reaction of the appropriate vanillin with the requisite alcohol of the formula $R^2$-OH, analogous to the procedure of Preparation 1, afforded the following compound :

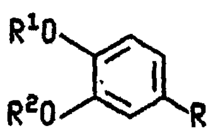

| Prep.# | $R^1$ | R | $R^2$ | M.P. °C | M.W. | Mass Spec (M+) | Analysis | | | |
|---|---|---|---|---|---|---|---|---|---|---|
| | | | | | | | Calculated (%) | | Found (%) | |
| | | | | | | | C | H | C | H |
| 2 | $CH_3$ | 3-CHO | 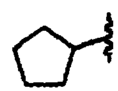 | oil | 220.3 | 220 | -- | -- | -- | -- |

PREPARATION 3

3-(Bicyclo[2.2.1]hept-2-yloxy-4-methoxy-α-methylbenzenemethanol

Added (108 mg, 2.85 mmol, 1.1 eq) sodium borohydride to a stirred solution of (675 mg, 2.59 mmol, 1.0 eq) (±)-3-methoxy-4-exo-norbornyloxy-acetophenone in 15 ml MeOH and 15 ml tetrahydrofuran. After 2 hours at room temperature, the reaction mixture was concentrated, poured into 200 ml of ethyl acetate, washed once with $H_2O$, once with brine, dried over $Na_2SO_4$, and then concentrated to yield 0.72 g of a clear oil. Silica gel chromatography eluting with 10% ethyl acetate/$CH_2Cl_2$ afforded 672 mg, 99%, of a dear oil. Mass spectra (M+): 262.

PREPARATION 4

Methyl 2-butyl-(4-hydroxyphenyl)-1H-benzimidazole-5-carboxlate

A mixture of (1.5 g, 6.98 mmol. 1.0 eq) methyl-3-nitro-4-chlorobenzoate and (760 mg, 6.98 mmol, 1.0 eq) 4-aminophenol in 30 ml dry dimethylsulfoxide was heated to reflux for 18 hours. The mixture was poured into 300 ml of $H_2O$, acidified to pH 5 and extracted once with ether. The resulting sludge was fittered through celite, and the filtrate layers separated. The aqueous layer was extracted with ether, and the ether extracts were combined, washed twice with $H_2O$, once with brine, dried over $MgSO_4$, and concentrated to give 3.0 g of an oil. Silica gel chromatography eluting with 30% ethyl acetate-hexane gave 850 mg, 42%, of a dark red gum.

A mixture of (850 mg, 2.95 mmol, 1.0 eq) of methyl 4-[(4-hydroxyphenyl)amino]-3-nitrobenzoate and 850 mg of 10% Pd/C in 40 ml ethyl acetate was placed on a Parr hydrogenation apparatus and shaken for 3 hours under 40 psi $H_2$. The mixture was filtered through celite, concentrated, and chromatographed on a silica gel column eluting with 40% ethyl acetate/hexane to yield 470 mg, 56%, of an off-white solid.

A mixture of (436 mg, 1.69 mmol, 1.0 eq) methyl 4-[4-hydroxyphenyl)amino]-2-aminobenzoate and 3 ml of valeric anhydride was heated to reflux for 3 hours. The reaction mixture was cooled to room temperature and flash chromatographed on a silica gel column eluting with 10% ethyl acetate/$CH_2Cl_2$ to give an oil, which was taken up in 15 ml MeOH and treated with 5 ml 1N NaOH for 1 hour. The mixture was poured into 300 ml of $H_2O$, acidified to pH 5 and extracted twice with ethyl acetate. The ethyl acetate extracts were combined, washed once with $H_2O$, once with brine, dried over $MgSO_4$ and concentrated to give 0.6 g of an oil. Flash chromatography on silica gel eluting with 2½% $CH_3OH/CH_2Cl_2$ gave 345 mg, 63%, of white foam. Mass Spectra: 324.2.

PREPARATION 5

2-Butyl-3-(4-hydroxyphenyl)benzimidazole

A mixture of (8.0 g, 51 mmol, 1.0 eq) 1-chloro-2-nitrobenzene and (5.64 g, 51 mmol, 1.0 eq) 4-aminophenol in 40 ml of dry dimethylsulfoxide was heated to reflux for 18 hours. The reaction mixture was cooled, poured into 400 ml of 0.1N HCl and 400 ml ethyl acetate, stirred, and filtered through celite. The filtrate layers were separated, and the aqueous layer was extracted with ethyl acetate. The ethyl acetate extracts were combined, washed twice with $H_2O$, once with brine, dried over $MgSO_4$, and concentrated to give 8 g of a dark oil. Silica gel chromatography eluting with 20% ethyl acetate/hexane gave 1.63 g, 14%, of a red solid.

A mixture of (1.6 g, 6.69 mmol, 1.0 eq) 4-N-(2-nitrophenyl)amino phenol and 800 mg of 10% Pd/C in 100 ml ethyl acetate was placed on a Parr hydrogenation apparatus and shaken under 50 psi $H_2$ for 3 hours. The mixture was filtered through celite, concentrated in vacuo, and chromatographed on a silica gel column eluting with 50% ethyl acetate/hexane to give 1.3 g, 94%, of an orange-yellow solid.

A mixture of (600 mg, 3.00 mmol, 1.0 eq) 4-N-(2-aminophenyl)amine phenol and 10 ml valeric anhydride was heated to reflux for 18 hours. The mixture was taken up in 50 ml of methanol, basified with 2N NaOH to pH 10, and stirred 1 hour at room temperature. The reaction mixture was then neutralized and extracted twice with ethyl acetate. The ethyl acetate extracts were combined, washed twice with $H_2O$, once with brine, dried over $MgSO_4$ and concentrated to give 1 g of an oil. Silica gel chromatography eluting with 2½% $CH_3OH$-$CH_2Cl_2$ gave 124 mg, 16%,solid. M.P.: 192-194°C.

PREPARATION 6

Methyl 1-(3-hydroxy-4-methoxyphenyl)-1H-benzimidazole-5-carboxylate

A mixture of (10 g, 74.5 mmoles) 5-amino-2-methoxyphenol and (13.3 g, 62 mmoles) methyl 3-nitro-4-chlorobenzoate in 50 ml of pyridine was stirred at room temperature overnight. The volatiles were removed in vacuo and the residue was dissolved in ethyl acetate and washed with dilute HCl, then dried over $MgSO_4$ and evaporated to give 12.7 g of crude product which was triturated with $CH_2Cl_2$ and filtered to give 3.9 g of purified methyl 4-N(4-methoxy-3-hydroxyphenyl)amino-3-nitrobenzoate.

A solution of 3.9 g of the above nitro compound in 75 ml of methanol and 60 ml of THF and 400 mg of 10% palladium on charcoal was shaken on a Parr shaker, at 40 psi $H_2$ for 5 hours. The catalyst was removed by filtration and the solvent evaporated in vacuo. The product methyl 4-N(4-methoxy-3-hydroxyphenyl)amino-3-aminobenzoate (3,4 g) was used without purification.

A mixture of 3.4 g of the above amine and 900 mg of ethyl formate in 25 ml of formic acid was heated at 100° overnight. The solvents were evaporated in vacuo to give 1.6 g of the title product.

PREPARATION 7

3-[2-[3-(Cyclopentyloxy)-4-methoxyphenyl]ethenyl]benzoic Acid

A mixture of (335 mg, 0.951 mmol, 1.0 eq) methyl, 3-[2-[3-(cyclopentyloxy)-4-methoxyphenyl]ethenyl]benzoate in 8 ml methanol and 1.9 ml (1.90 mmol, 2.0 eq) of 1N NaOH was heated to reflux for 1.5 hours. The reaction mixture was cooled to room temperature, concentrated in vacuo, poured into 100 ml $H_2O$, basified to pH 12, and washed once with ethyl acetate. The aqueous layer was acidified to pH 4 and extracted three times with ethyl acetate. The ethyl acetate extracts were combined, washed once with $H_2O$, once with brine, dried over $Na_2SO_4$, and concentrated to yield 295 mg of white waxy crystals. Recrystallization from ethyl acetate-hexane afforded 110 mg, 34%, of the cis isomer as white crystals. M.P.: 93-94°C. Elemental Analysis: Calc'd for $C_{21}H_{22}O_4$: Calc'd: C, 74.53; H, 6.55. Found: C, 74.32; H, 6.68.

## Claims

**1.** A compound of the formula

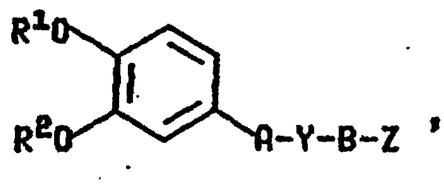

(I)

the racemic-diastereomeric mixtures and optical isomers of said compounds and the pharmaceutically acceptable salts thereof wherein

$R^1$ is selected from the group consisting of methyl, ethyl, difluoromethyl and trifluoromethyl;

$R^2$ is selected from the group consisting of ($C_1$-$C_6$)alkyl, alkoxyalkyl having 3 to 7 carbons in the alkoxy portion and 2 to 4 carbons In the alkyl portion, phenoxyalkyl having 2 to 6 carbons in the alkyl portion, ($C_3$-$C_7$)cycloalkyl, ($C_6$-$C_9$)polycycloalkyl, phenylalkyl having 1 to 8 carbons in the alkyl portion, phenylaminoalkyl having 2 to 6 carbons in the alkyl portion and the amino may be optionally substituted with ($C_1$-$C_4$) alkyl and indanyl,

where the alkyl portion of said alkyl, phenoxyalkyl, cycloalkyl, polycycloalkyl, phenylalkyl and indanyl may optionally be substituted with one or more fluorine atoms, -OH or ($C_1$-$C_4$)alkoxy,

and the aryl portion of said phenylalkyl, phenoxyalkyl and indanyl may optionally be substituted with ($C_1$-$C_4$) alkyl, ($C_1$-$C_4$)alkoxy or halogen;

A and B are independently selected from the group consisting of a covalent bond, optionally substituted ($C_1$-$C_6$) alkylene, optionally substituted ($C_2$-$C_6$)alkenyl and optionally substituted phenylene,

where said optionally substituted alkylene may be monosubstituted and each substituent is selected from the group consisting of oxo, ($C_1$-$C_4$)alkoxy, $CO_2R^6$ and hydroxy.

said optionally substituted alkenyl may be monosubstituted with ($C_1$-$C_4$)alkoxy or $CO_2R^6$, and

said optionally substituted phenylene may be monosubstituted with ($C_1$-$C_4$)alkoxy, $CO_2R^6$ or hydroxy.

wherein $R^6$ is hydrogen or ($C_1$-$C_4$)alkyl;

Y is selected from the group consisting of a covalent bond, O, $NR^6$ and S wherein $R^6$ is as defined above;

Z is selected from the group consisting of

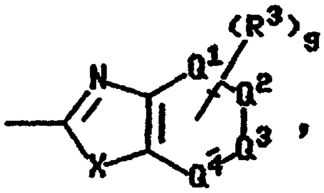 , 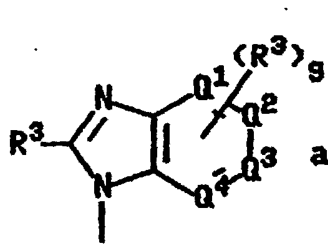 and 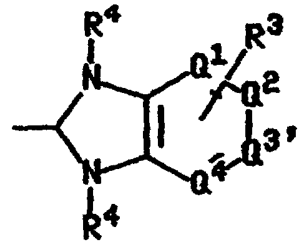

where $Q^1$, $Q^2$, $Q^3$, and $Q^4$ are CH ;
X is $NR^4$ ;
g is an integer from 1 to 4;
each $R^3$ is independently selected from the group consisting of hydrogen, halogen, $(C_1\text{-}C_6)$alkyl, $CH(R^7)CO_2R^4$; $(C_1\text{-}C_6)$alkoxy, $CO_2R^4$, $CONR^4R^5$, CONHOH, $CH_2NR^4R^5$, $NR^4R^5$, nitro, hydroxy, CN, $SO_3H$, phenylalkyl having 1 to 4 carbons in the alkyl portion, $SO_2NR^4R^5$, $N(SO_2R^6)_2$ and $NHSO_2R^6$,

where $R^4$ for each occurrence is independently selected from the group consisting of hydrogen, $(C_1\text{-}C_6)$alkyl, phenyl optionally substituted with $(C_1\text{-}C_4)$alkyl or halogen, $CH(R^7)CO_2R^6$, $(C_3\text{-}C_7)$cycloalkyl, phenylalkyl having 1 to 4 carbons in the alkyl portion and dialkylaminoalkyl having a total of 5 carbons in the dialkylamino portion and having 2 to 5 carbons in the alkyl portion where $R^6$ is as defined above,

$R^5$ for each occurrence is independently selected from the group consisting of hydrogen, $(C_1\text{-}C_6)$alkyl, $(C_3\text{-}C_7)$cycloalkyl, phenylalkyl having 1 to 4 carbons in the alkyl portion, phenyl, pyridyl, pyrimidyl, thiazolyl and oxazolyl,

or $R^4$ and $R^5$ are taken together with the nitrogen to which they are attached and form an optionally substituted saturated or unsaturated 5- or 6-membered ring, a saturated or unsaturated 6-membered heterocyclic ring containing two heteroatoms, or a quinoline ring optionally substituted with fluoro,

where said optionally substituted saturated or unsaturated 5- or 6-membered ring may be mono- or di-substituted and each substituent is independently selected from the group consisting of alkyl having 1 to 4 carbons, $CO_2R^7$ wherein $R^7$ is as defined below, $CONH_2$, $CON(CH_3)_2$, oxo, hydroxy, $NH_2$ and $N(CH_3)_2$, and said saturated or unsaturated 6-membered heterocyclic ring containing two heteroatoms has the second heteroatom selected from the group consisting of O, S, NH, $NCH_3$, $NCOCH_3$ and $NCH_2Ph$;

$R^7$ for each occurrence is independently selected from the group consisting of hydrogen and $(C_1\text{-}C_4)$alkyl;

and $R^8$ is selected from the group consisting of $(C_1\text{-}C_6)$alkyl, $(C_3\text{-}C_7)$cycloalkyl, phenyl and phenyalkyl having 1 to 4 carbons in the alkyl portion.

**2.** A compound or a pharmaceutically acceptable salt thereof according to claim 1, wherein

$R^1$ is selected from the group consisting of methyl and difluoromethyl;

$R^2$ is selected from the group consisting of $(C_3\text{-}C_7)$cycloalkyl, $(C_6\text{-}C_9)$polycycloalkyl, phenylalkyl having 1 to 8 carbons in the alkyl portion and phenoxyalkyl having 2 to 6 carbons in the alkyl portion;

A is selected from the group consisting of a covalent bond, $(C_1\text{-}C_5)$alkylene and $(C_2\text{-}C_5)$alkenyl;

B is selected from the group consisting of a covalent bond, phenylene optionally substituted with $(C_1\text{-}C_4)$alkoxy, $(C_1\text{-}C_5)$alkylene and $(C_2\text{-}C_5)$alkenyl;

Y is selected from the group consisting of a covalent bond, O and $NR^6$;

Z is selected from the group consisting of

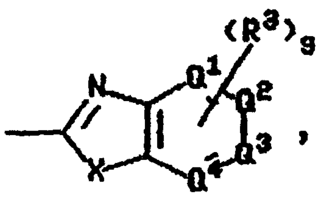 , 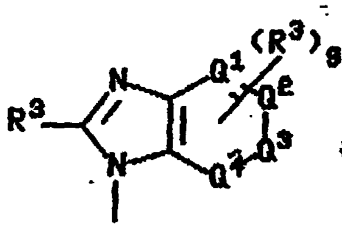 and 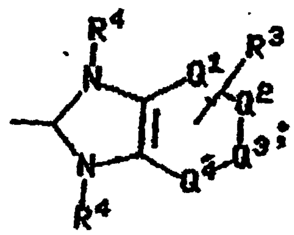

each $R^3$ is independently selected from the group consisting of hydrogen, halogen, $(C_1\text{-}C_6)$alkyl, $CH(R^7)CO_2R^4$,

$(C_1-C_6)$alkoxy, $CO_2R^4$, $CONR^4R^5$, nitro, hydroxy, $N(SO_2R^8)_2$ and $NHSO_2R^8$,

where $R^4$ for each occurrence is independently selected from the group consisting of hydrogen and $(C_1-C_6)$ alkyl and

$R^5$ is selected from the group consisting of hydrogen and $(C_1-C_6)$alkyl.

**3.** A compound or a pharmaceutically acceptable salt thereof according to claim 2, wherein
$R^2$ is selected from the group consisting of $(C_3-C_7)$cycloalkyl, $(C_6-C_9)$polycycloalkyl and phenylalkyl having 1 to 8 carbons in the alkyl portion;
A is selected from the group consisting of a covalent bond and methytene;
B is selected from the group consisting of a covalent bond, methylene and phenylene;
Y is selected from the group consisting of a covalent bond and O; and
Z is selected from the group consisting

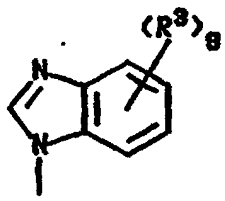

and

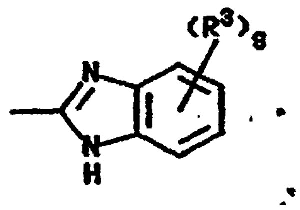

**4.** A compound or a pharmaceutically acceptable salt thereof according to claim 3, wherein A, B and Y are a covalent bond; and Z is selected from the group consisting of

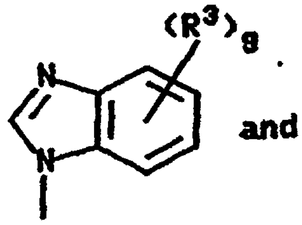

and

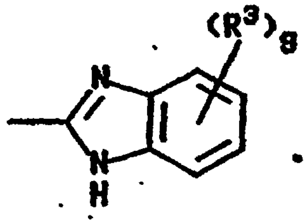

**5.** A compound or a pharmaceutically acceptable salt thereof according to claim 2 wherein
A is methylene;
B is phenylene;
Y is O; and
Z is

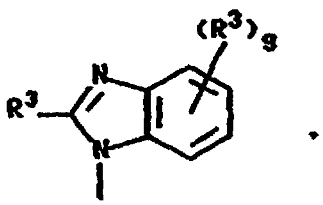

**6.** A pharmaceutical composition comprising a compound of the formula (I), as claimed in any one of claims 1 to 5, or a pharmaceutically acceptable salt thereof, and a pharmaceutically acceptable diluent or carrier.

**7.** A compound of the formula (I), or a pharmaceutically acceptable salt or composition thereof, as claimed in any one of claims 1 to 5 and 6, respectively, for use as a medicament.

**8.** The use of a compound of the formula (I), or a pharmaceutically acceptable salt or composition thereof, as claimed

in any one of claims 1 to 5 and 6, respectively, in the manufacture of a medicament for the inhibition of phosphodiesterase (IV) in a mammal.

**9.** The use of a compound of the formula (I), or a pharmaceutically acceptable salt or composition thereof, as claimed in any one of claims 1 to 5 and 6, respectively, in the manufacture of a medicament for the treatment of an inflammatory condition in a mammal.

**10.** The use of a compound of the formula (I), or a pharmaceutically acceptable salt or composition thereof, as claimed in any one of claims 1 to 5 and 6, respectively, in the manufacture of a medicament for the treatment of AIDS, asthma, arthritis, bronchitis, chronic obstructive pulmonary disease, psoriasis, allergic rhinitis, dermatitis or shock in a mammal.

**Patentansprüche**

**1.** Verbindung der Formel

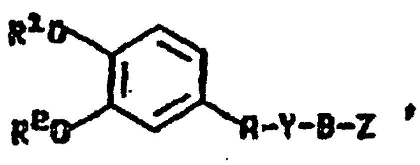

(I)

racemisch-diastereomere Mischungen und optische Isomere dieser Verbindungen und die pharmazeutisch annehmbaren Salze davon, worin

$R^1$ ausgewählt ist aus der Gruppe bestehend aus Methyl-, Ethyl-, Difluormethyl- und Trifluormethylresten;

$R^2$ ausgewählt ist aus der Gruppe bestehend aus $(C_1\text{-}C_6)$-Alkyl-, Alkoxyalkylresten mit 3 bis 7 Kohlenstoffatomen im Alkoxyanteil und 2 bis 4 Kohlenstoffatomen im Alkylanteil, Phenoxyalkylresten mit 2 bis 6 Kohlenstoffatomen im Alkylanteil, $(C_3\text{-}C_7)$-Cycloalkyl-, $(C_6\text{-}C_9)$-Polycycloalkyl-, Phenylalkylresten, mit 1 bis 8 Kohlenstoffatomen im Alkylanteil, Phenylaminoalkylresten mit 2 bis 6 Kohlenstoffatomen im Alkylanteil, wobei der Aminorest gegebenenfalls mit $(C_1\text{-}C_4)$-Alkylresten und Indanylresten substituiert sein kann,

wobei der Alkylanteil der Alkyl-, Phenoxyalkyl-, Cycloalkyl-, Polycycloalkyl-, Phenylalkyl- und Indanylreste gegebenenfalls mit einem oder mehreren Fluoratomen, -OH oder $(C_1\text{-}C_4)$-Alkoxyresten substituiert sein kann und

der Arylanteil der Phenylalkyl-, Phenoxyalkyl- und Indanylreste gegebenenfalls mit $(C_1\text{-}C_4)$-Alkyl-, $(C_1\text{-}C_4)$-Alkoxy- oder Halogenresten substituiert sein kann;

A und B unabhängig ausgewählt sind aus der Gruppe bestehend aus einer kovalenten Bindung, gegebenenfalls substituierten $(C_1\text{-}C_5)$-Alkylenresten, gegebenenfalls substituierten $(C_2\text{-}C_5)$-Alkenylresten und gegebenenfalls substituierten Phenylenresten,

wobei der gegebenenfalls substituierte Alkylenrest einfach substituiert sein kann und jeder Substituent ausgewählt ist aus der Gruppe bestehend aus Oxo-, $(C_1\text{-}C_4)$-Alkoxy-, $CO_2R^6$- und Hydroxyresten,

der gegebenenfalls substituierte Alkenylrest einfach substituiert sein kann mit $(C_1\text{-}C_4)$-Alkoxy oder $CO_2R^6$ und der gegebenenfalls substituierte Phenylenrest einfach substituiert sein kann mit $(C_1\text{-}C_4)$-Alkoxy-, $CO_2R^6$oder Hydroxyresten,

wobei $R^6$ Wasserstoff oder ein $(C_1\text{-}C_4)$-Alkylrest ist;

Y ausgewählt ist aus der Gruppe bestehend aus einer kovalenten Bindung, O, $NR^6$ und S, wobei $R^6$ wie oben definiert ist;

Z ausgewählt ist aus der Gruppe bestehend aus

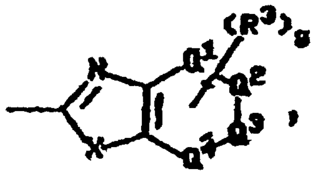, 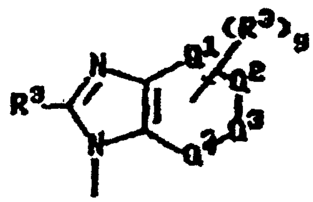 und 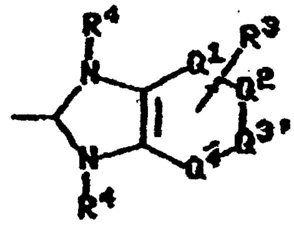

worin $Q^1$, $Q^2$, $Q^3$ und $Q^4$ CH sind;
X $NR^4$ ist;
g eine ganze Zahl von 1 bis 4 ist;
jeder Rest $R^3$ unabhängig ausgewählt ist aus der Gruppe bestehend aus Wasserstoff, Halogen, $(C_1\text{-}C_6)$-Alkyl, $CH(R^7)CO_2R^4$, $(C_1\text{-}C_6)$-Alkoxy, $CO_2R^4$, $CONR^4R^5$, CONHOH, $CH_2NR^4R^5$, $NR^4R^5$, Nitro, Hydroxy, CN, $SO_3H$, Phenylalkyl mit 1 bis 4 Kohlenstoffatomen im Alkylteil, $SO_2NR^4R^5$, $N(SO_2R^8)_2$ und $NHSO_2R^8$,

wobei $R^4$ bei jedem Vorkommen unabhängig ausgewählt ist aus der Gruppe bestehend aus Wasserstoff, $(C_1\text{-}C_6)$-Alkylresten, Phenylresten, die gegebenenfalls mit $(C_1\text{-}C_4)$-Alkylresten oder Halogen substituiert sind, $CH(R^7)CO_2R^8$, $(C_3\text{-}C_7)$ -Cycloalkyl-, Phenylalkylresten mit 1 bis 4 Kohlenstoffatomen im Alkylanteil und Dialkylaminoalkylresten mit insgesamt 5 Kohlenstoffatomen im Dialkylaminoanteil und mit 2 bis 5 Kohlenstoffatomen im Alkylanteil, wobei $R^6$ wie oben definiert ist, $R^5$ beim jedem Vorkommen unabhängig ausgewählt ist aus der Gruppe bestehend aus Wasserstoff, $(C_1\text{-}C_6)$-Alkyl-, $(C_3\text{-}C_7)$-Cycloalkyl-, Phenylalkylresten mit 1 bis 4 Kohlenstoffatomen im Alkylanteil, Phenyl-, Pyridyl-, Pyrimidyl-, Thiazolyl- und Oxazolylresten

oder $R^4$ und $R^5$ zusammen mit dem Stickstoffatom, an das sie gebunden sind, einen gegebenenfalls substituierten gesättigten oder ungesättigten 5- oder 6-gliedrigen Ring, einen gesättigten oder ungesättigten 6-gliedrigen heterocyclischen Ring, der zwei Heteroatome enthält, oder einen Chinolinring, der gegebenenfalls mit Fluor substituiert ist, bilden können,

wobei der gegebenenfalls substituierte gesättigte oder ungesättigte 5- oder 6-gliedrige Ring mono- oder disubstituiert sein kann und jeder Substituent unabhängig ausgewählt ist aus der Gruppe bestehend aus Alkylresten mit 1 bis 4 Kohlenstoffatomen, $CO_2R^7$, wobei $R^7$ wie unten definiert ist, $CONH_2$, $CON(CH_3)_2$, Oxo, Hydroxy, $NH_2$ und $N(CH_3)_2$ und bei dem gesättigten oder ungesättigten 6-gliedrigen heterocyclischen Ring, der zwei Heteroatome enthält, das zweite Heteroatom ausgewählt ist aus der Gruppe bestehend aus O, S, NH, $NCH_3$, $NCOCH_3$ und $NCH_2Ph$;
$R^7$ bei jedem Vorkommen unabhängig ausgewählt ist aus der Gruppe bestehend aus Wasserstoff und $(C_1\text{-}C_4)$ -Alkylresten und
$R^8$ ausgewählt ist aus der Gruppe bestehend aus $(C_1\text{-}C_6)$-Alkyl-, $(C_3\text{-}C_7)$-Cycloalkyl-, Phenyl- und Phenylalkylresten mit 1 bis 4 Kohlenstoffatomen im Alkylanteil.

**2.** Verbindung oder pharmazeutisch annehmbares Salz nach Anspruch 1, wobei
$R^1$ ausgewählt ist aus der Gruppe bestehend aus Methylund Difluormethylresten;
$R^2$ ausgewählt ist aus der Gruppe bestehend aus $(C_3\text{-}C_7)$-Cycloalkyl-, $(C_6\text{-}C_9)$-Polycycloalkyl-, Phenylalkylresten mit 1 bis 8 Kohlenstoffatomen im Alkylanteil und Phenoxyalkylresten mit 2 bis 6 Kohlenstoffatomen im Alkylanteil;
A ausgewählt ist aus der Gruppe bestehend aus einer kovalenten Bindung, $(C_1\text{-}C_5)$-Alkylen- und $(C_2\text{-}C_5)$-Alkenylresten;
B ausgewählt ist aus der Gruppe bestehend aus einer kovalenten Bindung, Phenylenresten, die gegebenenfalls mit $(C_1\text{-}C_4)$-Alkoxy-, $(C_1\text{-}C_5)$-Alkylen- und $(C_2\text{-}C_5)$-Alkenylresten substituiert sind;
Y ausgewählt ist aus der Gruppe bestehend aus einer kovalenten Bindung, O und $NR^6$;
Z ausgewählt ist aus der Gruppe bestehend aus

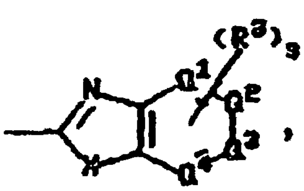, 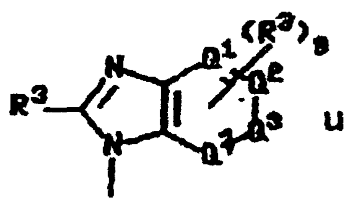 und 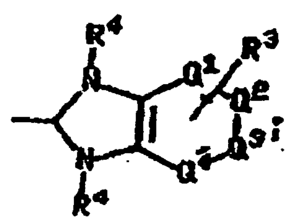

jeder Rest $R^3$ unabhängig ausgewählt ist aus der Gruppe bestehend aus Wasserstoff, Halogen, ($C_1$-$C_6$)-Alkyl, $CH(R^7)CO_2R^4$, ($C_1$-$C_6$)-Alkoxy, $CO_2R^4$, $CONR^4R^5$, Nitro, Hydroxy, $N(SO_2R^8)_2$ und $NHSO_2R^8$, wobei $R^4$ bei jedem Vorkommen unabhängig ausgewählt ist aus der Gruppe bestehend aus Wasserstoff und ($C_1$-$C_6$)-Alkylresten und $R^5$ ausgewählt ist aus der Gruppe bestehend aus Wasserstoff und ($C_1$-$C_6$)-Alkylresten.

**3.** Verbindung oder pharmazeutisch annehmbares Salz nach Anspruch 2, wobei
$R^2$ ausgewählt ist aus der Gruppe bestehend aus ($C_3$-$C_7$)-Cycloalkyl-, ($C_6$-$C_9$)-Polycycloalkyl- und Phenylalkylresten mit 1 bis 8 Kohlenstoffatomen im Alkylanteil;
A ausgewählt ist aus der Gruppe bestehend aus einer kovalenten Bindung und einem Methylenrest,
B ausgewählt ist aus der Gruppe bestehend aus einer kovalenten Bindung, einem Methylen- und Phenylenrest;
Y ausgewählt ist aus der Gruppe bestehend aus einer kovalenten Bindung und O und
Z ausgewählt ist aus der Gruppe bestehend aus

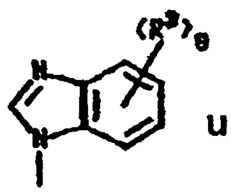

und

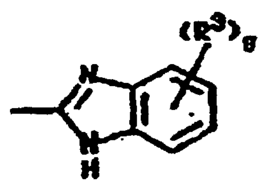

**4.** Verbindung oder pharmazeutisch annehmbares Salz nach Anspruch 3, wobei A, B und Y eine kovalente Bindung sind und Z ausgewählt ist aus der Gruppe bestehend aus

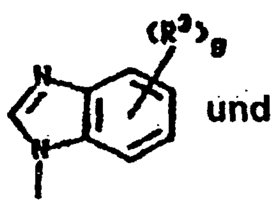

und

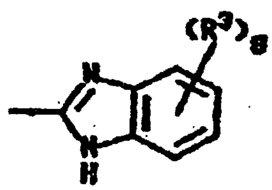

**5.** Verbindung oder pharmazeutisch annehmbares Salz nach Anspruch 2, wobei
A ein Methylenrest ist;
B ein Phenylenrest ist;
Y O ist und
Z

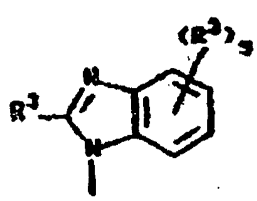

ist.

**6.** Pharmazeutische Zusammensetzung enthaltend eine Verbindung der Formel (I) wie in einem der Ansprüche 1 bis 5 beansprucht oder ein pharmazeutisch annehmbares Salz davon und ein pharmazeutisch annehmbares Verdünnungsmittel oder einen pharmazeutisch annehmbaren Träger.

**7.** Verbindung der Formel (I) oder ein pharmazeutisch annehmbares Salz oder eine Zusammensetzung davon nach einem der Ansprüche 1 bis 5 bzw. 6 zur Verwendung als Arzneimittel.

**8.** Verwendung einer Verbindung der Formel (I) oder eines pharmazeutisch annehmbaren Salzes oder einer Zusammensetzung davon nach einem der Ansprüche 1 bis 5 bzw. 6 zur Herstellung eines Arzneimittels zur Hemmung von Phosphodiesterase (IV) bei einem Säugetier.

**9.** Verwendung einer Verbindung der Formel (I) oder eines pharmazeutisch annehmbaren Salzes oder einer Zusammensetzung davon nach einem der Ansprüche 1 bis 5 bzw. 6 zur Herstellung eines Arzneimittels zur Behandlung eines entzündlichen Zustandes bei einem Säugetier.

**10.** Verwendung einer Verbindung der Formel (I) oder eines pharmazeutisch annehmbaren Salzes oder einer Zusammensetzung davon nach einem der Ansprüche 1 bis 5 bzw. 6 zur Herstellung eines Arzneimittels zur Behandlung von AIDS, Asthma, Arthritis, Bronchitis, chronisch obstruktiver Lungenkrankheit, Psoriasis, allergischer Rhinitis, Dermatitis oder Schock bei einem Säugetier.

**Revendications**

**1.** Composé de formule

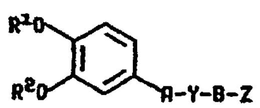

(I)

mélanges racémiques-diastéréoisomères et isomères optiques desdits composés et sels pharmaceutiquement acceptables de ceux-ci,
formule dans laquelle
$R^1$ est choisi dans le groupe constitué par les radicaux méthyle, éthyle, difluorométhyle et trifluorométhyle ;
$R^2$ est choisi dans le groupe constitué par les radicaux alkyle en $C_1$-$C_6$, alcoxyalkyle comportant 3 à 7 atomes de carbone dans la partie alcoxy et 2 à 4 atomes de carbone dans la partie alkyle, phénoxyalkyle comportant 2 à 6 atomes de carbone dans la partie alkyle, cycloalkyle en $C_3$-$C_7$, polycycloalkyle en $C_8$-$C_9$, phénylalkyle comportant 1 à 8 atomes de carbone dans la partie alkyle, phénylaminoalkyle comportant 2 à 6 atomes de carbone dans la partie alkyle et le radical amino pouvant être éventuellement substitué par un radical alkyle en $C_1$-$C_4$ et indanyle,

où la partie alkyle dudit radical alkyle, phénoxyalkyle, cycloalkyle, polycycloalkyle, phénylalkyle et indanyle peut être éventuellement substituée par un ou plusieurs atomes de fluor ou radicaux -OH ou alcoxy en $C_1$-$C_4$, et

la partie aryle desdits radicaux phénylalkyle, phénoxyalkyle et indanyle peut être éventuellement substituée par des radicaux alkyle en $C_1$-$C_4$, alcoxy en $C_1$-$C_4$ ou halogéno ;

A et B sont choisis, indépendamment l'un de l'autre, dans le groupe constitué par une liaison covalente, un radical alkylène en $C_1$-$C_5$ éventuellement substitué, alcényle en $C_2$-$C_5$ éventuellement substitué et phénylène éventuellement substitué,

où ledit radical alkylène éventuellement substitué peut être mono-substitué et chacun des substituants est choisi dans le groupe constitué par les radicaux oxo, alcoxy en $C_1$-$C_4$, $CO_2R^6$ et hydroxy, ledit radical alcényle éventuellement substitué peut être mono-substitué par un radical alcoxy en $C_1$-$C_4$ ou $CO_2R^6$, et

ledit radical phénylène éventuellement substitué peut être mono-substitué par un radical alcoxy en $C_1$-$C_4$, $CO_2R^6$ ou hydroxy,

dans lequel $R^6$ est un atome d'hydrogène ou un radical alkyle en $C_1$-$C_4$ ;

Y est choisi dans le groupe constitué par une liaison covalente ou un radical O, $NR^6$ et S, où $R^6$ est tel que défini ci-dessus ;
Z est choisi dans le groupe constitué par

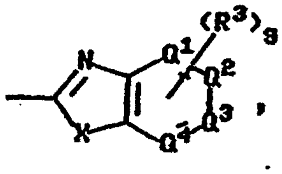 , 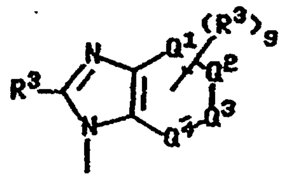 et 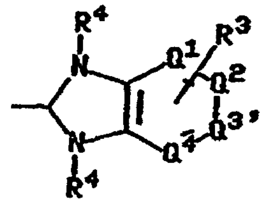

où $Q^1$, $Q^2$, $Q^3$ et $Q^4$ sont CH ;
X est $NR^4$ ;
g est un nombre entier de 1 à 4 ;
chaque $R^3$ est choisi, indépendamment des autres, dans le groupe constitué par un atome d'hydrogène ou d'halogène ou un radical alkyle en $C_1$-$C_6$, $CH(R^7)CO_2R^4$, alcoxy en $C_{1-6}$, $CO_2R^4$, $CONR^4R^5$, CONHOH, $CH_2NR^4R^5$, $NR^4R^5$, nitro, hydroxy, CN, $SO_3H$, phénylalkyle comportant 1 à 4 atomes de carbone dans la partie alkyle, $SO_2NR^4R^5$, $N(SO_2R^8)_2$ et $NHSO_2R^8$,

où $R^4$, à chaque fois qu'il apparaît, est choisi indépendamment des autres dans le groupe constitué par les atomes d'hydrogène et les radicaux alkyle en $C_1$-$C_6$, phényle éventuellement substitué par un groupe alkyle en $C_1$-$C_4$ ou halogéno, $CH(R^7)CO_2R^8$, cycloalkyle en $C_3$-$C_7$, phénylalkyle comportant 1 à 4 atomes de carbone dans la partie alkyle et dialkylaminoalkyle comportant un total de 5 atomes de carbone dans la partie dialkylamino et comportant 2 à 5 atomes de carbone dans la partie alkyle, où $R^8$ est tel que défini ci-dessus,

$R^5$, à chaque fois qu'il apparaît, est choisi indépendamment des autres dans le groupe constitué par les atomes d'hydrogène et les radicaux alkyle en $C_1$-$C_6$, cycloalkyle en $C_3$-$C_7$, phénylalkyle comportant 1 à 4 atomes de carbone dans la partie alkyle, phényle, pyridyle, pyrimidyle, thiazolyle et oxazolyle,

ou $R^4$ et $R^5$ sont pris conjointement avec l'atome d'azote auquel ils sont fixés et forment un noyau à 5 ou 6 chaînons, saturé ou insaturé, éventuellement substitué, un noyau hétérocyclique à 6 chaînons, saturé ou insaturé, contenant deux hétéroatomes, ou un noyau quinoléine éventuellement substitué par un radical fluoro, ledit noyau à 5 ou 6 chaînons, saturé ou insaturé, éventuellement substitué, pouvant être mono- ou di-substitué et chaque substituant étant indépendamment des autres choisi dans le groupe constitué par les radicaux alkyle comportant 1 à 4 atomes de carbone, $CO_2R^7$ où $R^7$ est tel que défini ci-dessous, $CONH_2$, $CON(CH_3)_2$, oxo, hydroxy, $NH_2$ et $N(CH_3)_2$, et ledit noyau hétérocyclique à 6 chaînons, saturé ou insaturé, contenant deux hétéroatomes ayant le second hétéroatome choisi dans le groupe constitué par O, S, NH, $NCH_3$, $NCOCH_3$ et $NCH_2Ph$ ;

$R^7$, à chaque fois qu'il apparaît, est choisi indépendamment des autres dans le groupe constitué par les atomes d'hydrogène et les radicaux alkyle en $C_1$-$C_4$ ;
et $R^8$ est choisi dans le groupe constitué par les radicaux alkyle en $C_1$-$C_6$, cycloalkyle en $C_3$-$C_7$, phényle et phénylalkyle comportant 1 à 4 atomes de carbone dans la partie alkyle.

**2.** Composé, ou sel pharmaceutiquement acceptable de ce composé, selon la revendication 1, dans lequel
$R^1$ est choisi dans le groupe constitué par les radicaux méthyle et difluorométhyle ;
$R^2$ est choisi dans le groupe constitué par les radicaux cycloalkyle en $C_3$-$C_7$, polycycloalkyle en $C_8$-$C_9$, phénylalkyle comportant 1 à 8 atomes de carbone dans la partie alkyle et phénoxyalkyle comportant 2 à 6 atomes de carbone dans la partie alkyle ;
A est choisi dans le groupe constitué par une liaison covalente, un radical alkylène en $C_1$-$C_5$ et un radical alcényle en $C_2$-$C_5$ ;
B est choisi dans le groupe constitué par une liaison covalente, un radical phénylène éventuellement substitué par un groupe alcoxy en $C_1$-$C_4$, alkylène en $C_1$-$C_5$ et alcényle en $C_2$-$C_5$ ;
Y est choisi dans le groupe constitué par une liaison covalente, O et $NR^6$ ;
Z est choisi dans le groupe constitué par

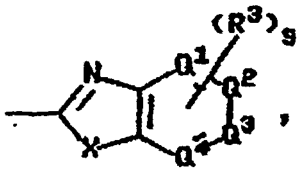,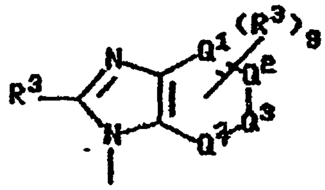 et 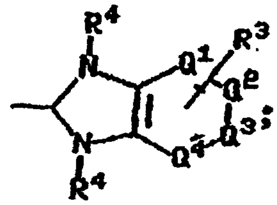;

où chaque $R^3$ est choisi, indépendamment des autres, dans le groupe constitué par un atome d'hydrogène ou d'halogène ou un radical alkyle en $C_1$-$C_6$, $CH(R^7)CO_2R^4$, alcoxy en $C_{1-6}$, $CO_2R^4$, $CONR^4R^5$, nitro, hydroxy, $N(SO_2R^8)_2$ et $NHSO_2R^8$,

où $R^4$, à chaque fois qu'il apparaît, est choisi indépendamment des autres dans le groupe constitué par les atomes d'hydrogène et les radicaux alkyle en $C_1$-$C_6$ et

$R^5$ est choisi dans le groupe constitué par un atome d'hydrogène et un groupe alkyle en $C_1$-$C_6$.

3. Composé, ou sel pharmaceutiquement acceptable de ce composé, selon la revendication 2, dans lequel $R^2$ est choisi dans le groupe constitué par les radicaux cycloalkyle en $C_3$-$C_7$, polycycloalkyle en $C_8$-$C_9$, phénylalkyle comportant 1 à 8 atomes de carbone dans la partie alkyle ;
A est choisi dans le groupe constitué par une liaison covalente et un radical méthylène ;
B est choisi dans le groupe constitué par une liaison covalente et un radical méthylène ou phénylène ;
Y est choisi dans le groupe constitué par une liaison covalente et O ; et
Z est choisi dans le groupe constitué par

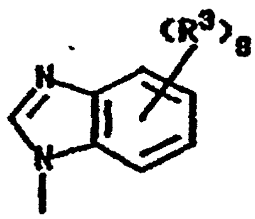 et 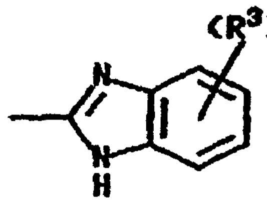.

4. Composé, ou sel pharmaceutiquement acceptable de ce composé, selon la revendication 3, dans lequel A, B et Y sont une liaison covalente ; et Z est choisi dans le groupe constitué par

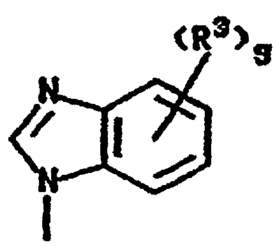 et 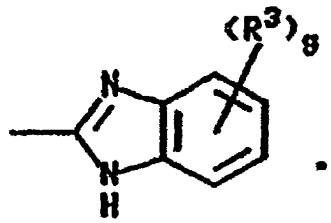.

5. Composé, ou sel pharmaceutiquement acceptable de ce composé, selon la revendication 2, dans lequel
A est un radical méthylène ;
B est un radical phénylène ;
Y est O ; et
Z est

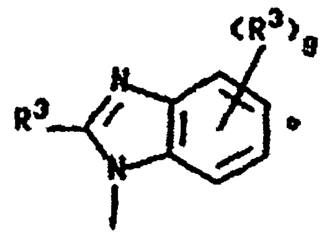

**6.** Composition pharmaceutique comprenant un composé de formule (I), selon l'une quelconque des revendications 1 à 5, ou un sel pharmaceutiquement acceptable de ce composé, et un diluant ou un véhicule pharmaceutiquement acceptable.

**7.** Composé de formule (I), ou sel ou composition pharmaceutiquement acceptable de ce composé, selon l'une quelconque des revendications 1 à 5 et 6, respectivement, à utiliser comme médicament.

**8.** Utilisation d'un composé de formule (I), ou d'un sel ou d'une composition pharmaceutiquement acceptable de ce composé, selon l'une quelconque des revendications 1 à 5 et 6, respectivement, dans la fabrication d'un médicament destiné à inhiber la phosphodiestérase (IV) chez un mammifère.

**9.** Utilisation d'un composé de formule (I), ou d'un sel ou d'une composition pharmaceutiquement acceptable de ce composé, selon l'une quelconque des revendications 1 à 5 et 6, respectivement, dans la fabrication d'un médicament destiné à traiter un état inflammatoire chez un mammifère.

**10.** Utilisation d'un composé de formule (I),ou d'un sel ou d'une composition pharmaceutiquement acceptable de ce composé, selon l'une quelconque des revendications 1 à 5 et 6, respectivement, dans la fabrication d'un médicament destiné à traiter le SIDA, l'asthme, l'arthrite, la bronchite, la bronchopneumopathie chronique obstructive, le psoriasis, les rhinites allergiques, l'eczéma ou le choc chez un mammifère.